# EUROPEAN PATENT SPECIFICATION

(11) **EP 1 740 668 B1**
(45) Date of publication and mention of the grant of the patent: **10.06.2015**
(21) Application number: 05736948.0
(22) Date of filing: 26.04.2005
(51) Int. Cl.: C09J 133/06, A61K 9/70, A61L 15/58, A61L 15/44, C08F 6/00

(54) **ADHESIVE FOR DERMAL PATCH AND PRODUCTION PROCESS THEREOF**
KLEBER FÜR HAUT APPLICATIONEN UND DESSEN HERSTELLUNG
ADHÉSIF POUR PATCH DERMIQUE ET SON PROCESSUS DE PRODUCTION

(30) Priority: 27.04.2004 JP 2004131080
(43) Date of publication of application: 10.01.2007
(73) Proprietor: SHOWA DENKO K.K., Tokyo 105-8518 (JP)
(72) Inventor: ISHII, Tetsuya, Production & Tech. Control Dept., Kawasaki-shi, Kanagawa 210-0867 (JP)
(74) Representative: Strehl Schübel-Hopf & Partner
(86) International application number: PCT/JP2005/008391
(87) International publication number: WO 2005/103184

(56) References cited:
- EP-A- 0 309 404
- US-B1- 6 552 141
- PATENT ABSTRACTS OF JAPAN vol. 018, no. 415 (C-1233), 4 August 1994 (1994-08-04) & JP 06 122707 A (NIPPON SHOKUBAI CO LTD), 6 May 1994 (1994-05-06)

## Description

### TECHNICAL FIELD

The present invention relates to a process for producing an adhesive comprising an acrylate-base polymer as a main ingredient used for dermal patch such as a percutaneous absorption-type preparation for external application, etc. More specifically, the present invention relates to a process for producing an acrylate-base polymer with less scatterings in the physical properties such as adhesion which is important as a dermal patch and capable of ensuring stable quality.

### BACKGROUND ART

Generally, a hydrous type dermal patch such as a poultice is formed by ionic crosslinking of sodium polyacrylate, polyacrylic acid, acrylic acid-sodium acrylate copolymer, crosslinked polyacrylic acid, etc. with an aluminum compound. In a case where the ionic crosslinking is not sufficient, an adhesive layer exudes to a non-woven fabric, etc. as a support, as well as "glue residue" occurs to skins giving an unpleasant feeling to a patient. Further, excess crosslinking hardens the adhesive layer to deteriorate adhesion, to result in a problem such as detachment from skins or worsening of percutaneous absorption of medicines. Accordingly, crosslinking between the aluminum compound and the acrylate-base polymer is extremely important in view of the quality.

As the aluminum compound used upon manufacture of hydrous type dermal patch, those of less water soluble are generally used. This is because crosslinking proceeds instantly in a case of using those of easily soluble in water, thereby making crosslinking not uniform. Accordingly, a method of gradually dissolving a sparingly soluble aluminum compound with an acid to conduct crosslinking is generally used. As the acid, use of an oxycarboxylic acid such as lactic acid or tartaric acid having hydroxyl group and carboxyl group in the molecule can increase the dissolution rate to improve the productivity (JP-A-60-226808). Accordingly, the amount of the aluminum compound and the amount of the oxycarboxylic acid are important for the quality of the of hydrous type dermal patch.

Further, in the composition of the hydrous type adhesive for the dermal patch, the blending amount of the acrylate-base polymer is larger next to water and the humectant such as glycerine. However, literatures considering the effects of the acrylate-base polymer on the extent of crosslinking between the polymer and an aluminum compound have been scarcely found in the past.

The present inventors have noted that the degree of the crosslinking varies greatly by strictly controlling the lots or the content of the aluminum compound and the oxycarboxylic acid, made earnest study on the manufacturing method of various acrylate-base polymers, analyzed impurities of the acrylate-base polymer with gas chromatography or liquid chromatography, and studied on the relation between them and the degree of crosslinking. It has been found in this course that β-hydroxypropionic acid (hydroacrylic acid) has an intense relation and the present invention has been accomplished.

EP-A-0309404 discloses the preparation of adhesive compositions for dermal patch applications comprising purifying the polymer via dialyse.

### DISCLOSURE OF THE INVENTION

The present invention is intended to provide a process for producing a dermal patch having a stable shape retainability, adhesion and percutaneous absorptivity of medicines, as a result of using an acrylate-base polymer with less content of β-hydroxypropionic acid (salt), thereby stabilizing the crosslinking reaction between carboxyl groups in the polymer and aluminum.

As a result of extensive investigations by taking account of those problems, the crosslinking rate between an aluminum compound and the polymer can be controlled easily by restricting the concentration of β-hydroxypropionic acid (salt) in an acrylate-base polymer and, as a result, hardness of the adhesive layer can be made constant, the adhesion is also stabilized and the reproducibility can also be ensured for the absorption of the medicines. Further, problems such as detachment also reduced to facilitate the quality control. Further, more safe adhesive for the dermal patch to be applied to a human body can be provided by restricting the concentration of β-hydroxypropionic acid (salt) in an acrylate-base polymer.

β-hydroxypropionic acid is formed from acrylic acid as shown below.

CH₂=CH-COOH + HOH → HO-CH₂CH₂-COOH

On the other hand, 100% reaction is not usually carried out in the polymerization and unreacted monomers always remain. Also the polymerization of acrylic acid is not the exception and residual acrylic acid is present as impurities in the polymer after polymerization. That is, β-hydroxypropionic acid is a compound that is inevitably contained in the manufactureing process of a polymer starting from acrylic acid in a case where a water-mediated process is present.

On the other hand, a less soluble aluminum compound is used as the crosslinking agent in the manufacturing process of a hydrous type dermal patch such as a cataplasm and is dissolved by using an oxycarboxylic acid such as lactic acid or tartaric acid having both hydroxyl group and carboxyl group in the molecule. It is to be noted that β-hydroxypropionic acid is also an oxycarboxylic acid.

However, it has been known that the crosslinking rate is rather decelerated when the oxycarboxylic acid is blended in a great amount. It is considered that the liquid pH is lowered to coagulate the polymer and that the oxycarboxylic acid masks aluminum ions by the chelating effect to hinder ionic bonding between the aluminum ions and the carboxylates of the polymer. Actually, tartaric acid or citric acid is generally used industrially as the chelating agent ("nyuumon kireto kagaku (introduction to chelate chemistry)" written by Keihei Ueno, from Nankodo).

That is, it is considered that when an oxycarboxylic acid other than the.oxycarboxylic acid added intentionally as a dissolution auxiliary agent for the aluminum compound is present, it promotes dissolution of aluminum making the crosslinking not uniform or, conversely, hinders ionic bonding between the aluminum ions and the ions of the polymer thereby making the crosslinking insufficient. As described above, the β-hydroxypropionic acid is also an oxycarboxylic acid and it is reasonably considered that this gives an undesired effect on the degree of crosslinking depending on the content thereof.

JP-A-6-56931 describes that the concentration of the residual monomer in the polymer after polymerization can be decreased by restricting β-hydroxypropionic acid (salt) in the acrylic acid monomer before polymerization. However, this is an invention that essentially concerns decrease of acrylic acid (salt) remaining in the polymer and intends to control the concentration of β-hydroxypropionic acid (salt) as a method of decreasing the acrylic acid. Since the present invention intends to enhance the accuracy of the crosslinking reaction between the aluminum compound and the acrylate-base polymer by controlling the concentration of β-hydroxypropionic acid (salt) in the polymer, this is essentially different from the invention in JP-A-6-56931.

That is, the present inventors have noted on the importance of β-hydroxypropionic acid (salt) contained by a small amount in the acrylate-base polymer which was not noted at all so far, intended to stabilize the crosslinking reaction between the aluminum compound and the acrylate-base polymer by restricting the content thereof and have accomplished the adhesive for the dermal patch capable of manufacturing the dermal patch of high quality.

The present invention provides the following.
[1] A process for producing an adhesive for a dermal patch comprising an acrylate-base polymer containing 5 mol% or more of acrylic acid and/or salt thereof as a monomer unit in which the content of β-hydroxypropionic acid and/or salt thereof is 5,000 ppm or less per solid content of the polymer and an aluminum compound, wherein a drying step of controlling the acrylate-base polymer to a temperature of 40 to 160°C or lower is included.
[2] A process for producing an adhesive for a dermal patch comprising an acrylate-base polymer containing 5 mol% or more of acrylic acid and/or salt thereof as a monomer unit in which the content of β-hydroxypropionic acid and/or salt thereof is 5,000 ppm or less per solid content of the polymer and an aluminum compound, wherein a step of drying the acrylate-base polymer is included, and the water content in the acrylate-base polymer before drying step is controlled to 40% or less.
[3] The process for producing an adhesive for a dermal patch according to [2] above, wherein a dewatering step of dewatering the acrylate-base polymer previously with a water soluble organic solvent before the drying step is included.
[4] The process for producing an adhesive for a dermal patch according to any one of [1] to [3] above, wherein the step of reducing the content of β-hydroxypropionic acid and/or salt thereof by bringing the same into contact with a sub-critical fluid and/or super-critical fluid is included.
[5] The process for producing an adhesive for a dermal patch according to [4] above, wherein the sub-critical fluid and/or super-critical fluid is carbon dioxide.
[6] The process for producing an adhesive for a dermal patch according to [1] or [2] above, wherein an oxycarboxylic acid other than β-hydroxypropionic acid and an aluminum compound are blended after drying.
[7] The process for producing an adhesive for a dermal patch according to [1] or [2] above, wherein the acrylate-base polymer is sodium acrylate -N-vinyl acetamide copolymer.
[8] The process for producing an adhesive for a dermal patch according to [1] or [2] above, wherein the acrylate-base polymer has the repetitive unit represented by the formulae (1), (2) and (3) :

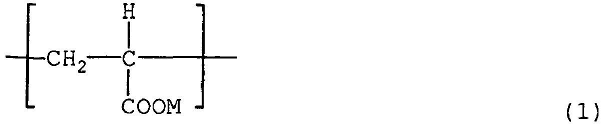

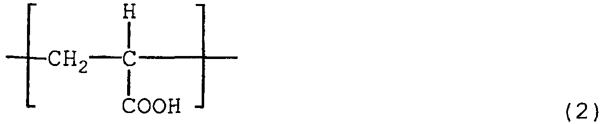

   -X- (3)

   wherein M represents NH₄⁺ or an alkali metal, and X represents N-vinylacetamide, N-methyl-N-vinylacetamide, N-vinyl pyrrolidone, methacrylic acid (salt), methoxy polyethyleneglycol(meth)acrylate, N,N- dimethylaminoethyl(meth)acrylate, or acrylamide unsaturated monomer, with a ratio of (1)/(2) being in a range from 100/0 to 0/100 (molar ratio) and total of (1) and (2)/(3) being in a range from 100/0 to 5/95.
[9] The process for producing an adhesive for a dermal patch according to [8] above, wherein M is sodium the ratio of the repetitive unit (1) and (2) is within a range of: (1)/(2)=20/80 to 80/20 (molar ratio) and the repetitive unit (3) is not contained.
[10] The process for producing an adhesive for a dermal patch according to [1] or [2] above, wherein the acrylate-base polymer is intra-molecularly crosslinked.
[11] A process for producing an adhesive used for a dermal patch comprising an acrylate-base polymer containing 5 mol% or more of acrylic acid and/or salt thereof as a monomer unit in which the content of β-hydroxypropionic acid and/or salt thereof is 5,000 ppm or less per solid content of the polymer, which includes:
   a) a step of preparing a polymer containing 5 mol% or more of acrylic acid and/or salt thereof as a monomer unit;
   b) a step of controlling a β-hydroxypropionic acid content in the polymer to 5,000 ppm or less;
   c) a step of adding to the polymer water, a controller for the cross-linking reaction rate and a crosslinker containing an aluminium compound; and
   d) a step of crosslinking the above mixture.
[12] The process for producing an adhesive used for a dermal patch according to [11] above, wherein the controller for cross-linking reaction rate is oxycarboxylic acid.
[13] The process for producing an adhesive used for a dermal patch according to [12] above, wherein the oxycarboxylic acid is other than β-hydroxypropionic acid.
[14] The process for producing an adhesive used for a dermal patch according to [11] above, wherein polyhydric alcohol is further blended in step c).

### TECHNICAL FIELD

The present invention is described in detail below.

In the adhesive for a dermal patch produced in the present invention, the content of β-hydroxypropionic acid (salt) in the acrylate-base polymer is essentially 5,000 ppm or less, preferably, 1,000 ppm or less, more preferably, 500 ppm or less and, most preferably, 100 ppm or less based on the solid content (Content of β-hydroxypropionic acid (salt) in the invention means the total content for β-hydroxypropionic acid and β-hydroxypropionic acid salt. In a case where the content of β-hydroxypropionic acid (salt) exceeds 5,000 ppm, since the crosslinking is conducted insufficiently or excessively in the resultant adhesive for the dermal patch and the hardness of the adhesive layer becomes instable, absorption of medicines is also instable as a result.

In the adhesive for a dermal patch produced in the present invention, it is preferred that the ratio of acrylate-base acid (salt) as the monomer unit constituting the acrylate-base polymer is 5 to 100 mol% (acrylic acid (salt) in the invention means total for acrylic acid and acrylate salts). In the invention, for developing the stabilization of the crosslinking reaction further, the ratio of the acrylic acid (salt) is, preferably, from 10 to 100 mol% and, further, from 40 to 100 mol%. In a case where the content of acrylic acid (salt) is less than 5 mol%, the crosslinking reaction with the aluminum compound takes much time and requires extension of the manufacturing time and increase for the concentration of the aluminum compound to increase the cost up which is not preferred. Within the range of the copolymerization ratio, while those of lower ratio of acrylic acid (salt) are also included, what is important is the content of β-hydroxypropionic acid (salt). Even when acrylic acid (salt) is charged by 15% upon charging for polymerization, because of poor copolymerizability with the mating monomer, it may eventually be contained sometimes only by 5% in the polymer. In this case, the unreacted acrylic acid (salt) sometimes amounts to 10%, which is converted into β-hydroxypropionic acid (salt).

In the adhesive for a dermal patch produced in the present invention, the acrylate-base polymer in which repetitive units are represented by the formulae (1), (2) and (3):

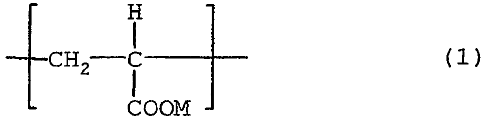

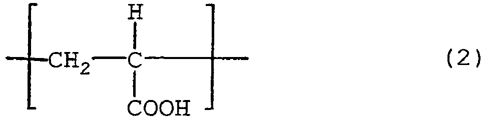

-X- (3)

wherein M represents NH₄⁺ or an alkali metal, and X represents other unsaturated monomer units than those of the formulae (1) and (2), with (1)/(2) = 100/0 to 0/100 (molar ratio), and total of (1) and (2)/(3) = 100/0 to 5/95 are particularly preferred.

The acrylate-base polymer includes, specifically, homopolymers of alkali metal salt such as sodium salt and potassium salt and ammonium salts of acrylic acid, copolymers of alkali metal salts such as sodium salt and potassium salt and ammonium salt of acrylic acid and acrylic acid, and homopolymers of acrylic acid, etc. They may be used in combination upon preparing the dermal patch. Among them, sodium salt or potassium salt is preferred and sodium salt is particularly preferred for obtaining a polymer having excellent physical properties.

Also, it is preferable that in the polymer, the acrylic acid be a sodium salt, that the ratio of repetitive units (1) and (2) above be in a range from 20/80 to 80/20 (molar ratio) and that no repetitive units (3) above be contained.

In the entire unsaturated monomers used in the adhesive for a dermal patch produced in the present invention, unsaturated monomers other than acrylic acid (salt) may also be used in an amount up to 95 mol%.

The unsaturated monomer other than acrylic acid (salt) includes, for example, acid group-containing hydrophilic unsaturated monomers such as methacrylic acid, maleic acid, maleic acid anhydride, fumaric acid, crotonic acid, itaconic acid, vinylsulfonic acid, styrene sulfonic acid, 2-(meth)acrylamide-2-methylpropane sulfonic acid, 2-(meth)acryloylethane sulfonic acid, and 2-(meth)acryloylpropane sulfonic acid and salts thereof; nonionic hydrophilic unsaturated monomers such as N-vinylacetamide, N-methyl-N-vinylacetamide, acrylamide, methacylamide, N-ethyl-(meth)acrylamide, N-n-propyl(meth)acrylamide, N-isopropyl(meth)acrylamide, N,N-dimethyl (meth) acrylamide, 2-hydroxylethyl (meth) acrylate, 2-hydroxypropyl(meth)acrylate, methoxypolyethylene glycol(meth)acrylate, polyethylene glycol mono(meth)acrylate, vinylpyridine, N-vinyl pyrrolidone, N-acryloyl piperidine, and N-acryloyl pyrrolidine; cationic hydrophilic unsaturated monomers such as N,N-dimethylaminoethyl(meth)acrylate, N,N-diethylaminoethyl(meth)acrylate, N,N-dimethylaminopropyl (meth)acrylate, N,N-dimethylaminopropyl(meth)acrylamide, and quaternary salts thereof; styrene, vinyl chloride, butadiene, isobutene, ethylene, propylene, stearyl (meth) acrylate, and lauryl (meth) acrylate, etc. and one or more of them selected from the groups described above can be used. Further, unsaturated monomers forming hydrophilic resins by hydrolysis for the functional groups such as methyl (meth) acrylate, ethyl(meth)acrylate, and vinyl acetate may also be used.

Among them, particularly, N-vinylacetamide, N-methyl-N-vinylacetamide, N-vinyl pyrrolidone, methacrylic acid (salt), methoxy polyethyleneglycol(meth)acrylate, N,N-dimethylaminoethyl(meth)acrylate, and acrylamide are preferred.

In the adhesive for a dermal patch produced in the present invention, the means for preparing the adhesive for the dermal patch in which the content of β-hydroxypropionic acid per solid content in the acrylate-base polymer is restricted to an amount of 5,000 ppm or less is not particularly limited.

While the polymerization method used in the adhesive for a dermal patch produced in the present invention can include known means in the public, for example, (a) a method by a radical polymerization initiator, (b) a method by radiation rays, (c) a method by a electron beam, (d) ultraviolet ray polymerization by photosensitizer and the like, polymerization by the radical polymerization initiator in (a) described above, is preferred for obtaining an acrylate-base polymer of high performance.

The polymerization method (a) includes known polymerization methods in the public, for example, various aqueous solution polymerization such as polymerization in mold form, polymerization by finely mincing the hydrous gel polymer and polymerization under pressing condition, reverse phase suspension polymerization, reverse phase emulsion polymerization, precipitation polymerization and bulk polymerization, the reverse phase suspension polymerization or aqueous solution polymerization is particularly preferred. The polymerization method may be any of continuous type, semi-batch type or batch type and can be practiced under any of the conditions at reduced pressure, elevated pressure or atmospheric pressure.

While polymerization may be bulk polymerization or precipitation polymerization, and a solution polymerization is preferred in view of the performance or the easy control for the polymerization. The solvent for the polymerization system has no particular restriction so long as this is a liquid to which the unsaturated monomer is soluble and includes, for example, water, methanol, ethanol, acetone, dimethylformamide, dimethylsulfoxide, etc., water or aqueous liquid being particularly preferred. Further, the concentration in a case of solution polymerization of the unsaturated monomer has no particular restriction and it may exceed the saturation concentration. In view of various physical properties and decrease of the residual monomer, it is within a range usually from 20 mass% to saturated concentration and, preferably, from 25 to 50 mass%. Further, upon polymerization, a chain transfer agent, and hydrophilic polymers such as starch, cellulose or derivatives thereof, polyvinyl alcohol, poly-N-vinyl acetamide crosslinked product, etc. may be added to the unsaturated monomer. The amount of them to be used is usually within 5 mass parts for the chain transfer agent and within 50 mass parts for the hydrophilic polymers. The chain transfer agent includes hypophosphites such as hypophosphorus acid and sodium hypophosphite, sulfur-containing compounds such as thioacetic acid, thioglycolic acid and mercapto ethanol, disulfides, halogenation products such as carbon tetrachloride and phosphites such as phosphorous acid and sodium phosphite.

The radical polymerization initiator used for the polymerization includes, for example, persulfates such as potassium persulfate, ammonium persulfate, and sodium persulfate; organic peroxides such as t-butyl hydroperoxides and cumene hydroperoxide; hydrogen peroxide; azo compounds, such as 2,2'-azobis(2-amidinopropane)dibasic acid salt; as well as known polymerization initiators such as chlorite, hypochlorite, ceric salt and permanganate and, among all, one or more of members selected from the group consisting of persulfates, hydrogen peroxide and azo compound are preferred. Further, in a case of using the oxidative radical polymerization initiator, a reducing agent such as hydrogen sulfite, iron, L-ascorbic acid, etc. may be used together. In a case of using the azo type polymerization initiator, etc. ultraviolet rays may also be used in combination. The radical polymerization initiators, etc. may be collectively added or added successively to the polymerization system, and the amount of use thereof is usually from 0.001 to 2 mol% and, preferably, from 0.01 to 1 mol% based on the unsaturated monomer.

In the adhesive for a dermal patch produced in the invention, the acrylate-base polymer may also be crosslinked. By the use of the crosslinked polymer, shape retainability and the solvent retainability of the dermal patch can be improved.

The crosslinking method is not particularly limited and includes, for example, (a) a method of obtaining an acrylate-base polymer and then further conducting post crosslinking with addition of a crosslinker during or after the polymerization, (b) radical crosslinking by a radical polymerization initiator, and (c) radiation ray-crosslinking with electron beam or the like. For improving the productivity, it is preferred (d) to conduct polymerization with addition of a predetermined amount of an internal crosslinker to the unsaturated monomer and then conducting crosslinking reaction simultaneously with or after polymerization.

As the internal crosslinker used for the preferred method (d), one or more of internal crosslinkers is used including, for example, N,N'-methylene bisacrylamide, (poly)ethyleneglycol di(meth)acrylate, (poly)propyleneglycol di(meth)acrylate, trimethylolpropane tri(meth)acrylate, trimethylolpropane di(meth)acrylate, (poly)ethyleneglycol di(β-acryloyloxypropionate), trimethylolpropane tri(β-acryloyloxypropionate), poly(meth)allyoxyalkane, (poly)ethylene glycol diglycidyl ether, ethyleneglycol, polyethyleneglycol, glycerine, pentaerythritol, ethylenediamine and polyethyleneimine. The amount of use thereof is usually from 0.005 to 5 mol%, preferably, from 0.01 to 1 mol% based on the unsaturated monomer. Among them, it is preferred to use the polymerizable internal crosslinker having two or more polymerizable unsaturated groups in one molecule.

As a method of decreasing β-hydroxypropionic acid (salt), since β-hydroxypropionic acid is present as a precursor of the monomer in acrylic acid, for instance, it may be considered a method of polymerization by using an acrylic acid (salt) with a less content of β-hydroxypropionic acid (salt), a method of decreasing the residual acrylic acid (salt) after polymerization by the catalyst preparation upon polymerization, or a method of suppressing the formation of or removing β-hydroxypropionic acid (salt) in the post-treatment for the polymer after polymerization.

Upon conducting polymerization by using the unsaturated monomer in the form of a solution, the acrylate-base polymer after the polymerization may be used as it is as an adhesive for the dermal patch but it is preferably dried with a view point of handability.

Drying is conducted by a known method. For example, a method of conducting polymerization at a high concentration thereby conducting drying and polymerization at the same time by the heat of polymerization may be adopted, or the obtained gel-like polymer may be further dried depending on the solid content after the polymerization. Further, in a case of conducting drying, methods, for example, of hot-air transfer type drying, material stirring type drying (using fluidized bed dryer, etc.), material transportation and settling type drying, cylinder drying, infrared-ray drying, microwave drying, superheated steam drying, etc. may be adopted.

The concentration of β-hydroxypropionic acid (salt) can be lowered by restricting the drying temperature as the drying condition. This is because β-hydroxypropionic acid is formed at a high temperature, in the presence of water even when the atmosphere is not alkaline. That is, drying at low temperature can decrease the ratio for the formation of β-hydroxypropionic acid from the residual acrylic acid in the polymer.

Accordingly, in the production method of the adhesive for a dermal patch of the present invention, drying step is usually conducted at 40 to 160°C, preferably, at 50 to 150°C and, more preferably, at 70 to 140°C. Outside the temperature range described above, the concentration of β-hydroxypropionic acid (salt) is increased. The drying time is arbitrarily determined depending on the water content and grain size of the polymer, drying temperature, etc.. It is usually within a range from 10 min to 12 hours.

Further, in view of the mechanism of forming β-hydroxypropionic acid, the concentration of β-hydroxypropionic acid (salt) can be lowered by restricting the water content in the acrylate-base polymer before drying as the drying condition.

The water content in the acrylate-base polymer before drying is usually 40% or less, more preferably, 30% or less and, further preferably, 10% or less. In a case where the water content in the acrylate-base polymer before drying is 40% or less, the drying temperature may exceed 160°C.

While the method of restricting the water content in the acrylate polymer before drying to 40% or less includes, for example, freeze drying or vacuum drying, the method of dewatering by using a water soluble organic solvent is simple and convenient. The water soluble organic solvent has no particular restriction so long as it is dissolved in water and includes, for example, alcohols such as methanol, ethanol, 1-propanol, 2-propanol, 1-butanol, 2-butanol, i-butylalcohol, t-butyl alcohol, 1-pentanol, 2-pentanol, 3-pentanol, isoamylalcohol, 4-methyl-2-pentanol, 1-heptanol, 2-heptanol, 1-octanol, 1-hexanol, 2-hexanol, 3-hexanol, and cyclohexanol; ketones such as acetone and methyl ethyl ketone, cellosolve, dioxane, dimethylformamide, N-methylpyrrolidone, and dimethylsulfoxide. Among them, methanol, ethanol and propanol are preferred in view of the dewatering efficiency and, particularly, ethanol is preferred in view of the safety to human bodies.

The water soluble organic solvent may be used as it is, may be used also as an aqueous solution and, further, plural water soluble organic solvents may be mixed and used. In this case, it is preferred to select the composition for the solvents so that the acrylate-base polymer is not dissolved out.

In the production method of the adhesive for a dermal patch of the present invention, β-hydroxypropionic acid (salt) can be decreased by bringing the acrylate-base polymer into contact with a sub-critical fluid and/or super-critical fluid. A method where the acrylate-base polymer is contacted with a sub-critical fluid and/or super-critical fluid is free from remaining of solvent, unlike a method using an organic solvent, and therefore is suitably used in consideration for the use of dermal patch which is applied to human body. It is preferred that the acrylate-base polymer to be contacted with a sub-critical fluid and/or super-critical fluid is preliminarily dried at temperature of 200°C or less. Although the generation rate of β-hydroxypropionic acid (salt) can be high depending on the drying temperature, generation of β-hydroxypropionic acid (salt) can be reduced by bringing the acrylate-base polymer into contact with a sub-critical fluid and/or super-critical fluid.

In the production method of the adhesive for a dermal patch of the present invention, the sub-critical fluid is a fluid under the condition of lower than the critical temperature and/or lower than the critical pressure. Further, the super-critical fluid is a fluid under the condition at a critical temperature or higher and at a critical pressure or higher. The kind of the fluid used specifically as the sub-critical fluid and the super-critical fluid is not particularly limited and various fluids known so far can be used. Further, plural kinds of the sub-critical fluids and the super-critical fluids may be used together. In a case of using the plural kinds of them together, they may be mixed and used simultaneously or may be used individually and successively.

As the sub-critical fluid and the super-critical fluid, at least one or more of fluids selected from the group consisting, for example, of carbon dioxide, carbon monoxide, nitrogen oxides (for example, dinitrogen monoxide), ammonia, saturated hydrocarbon (methane, ethane, propane, butane, etc.), unsaturated hydrocarbon (ethylene, propylene, butene, etc.), nitrogen, hydrogen, oxygen and halogenated hydrocarbon (chlorofluorocarbon gas, etc.) and dimethyl ether can be used preferably.

Among the fluids described above, with a view point of easy separation from the acrylate polymer after use, those substances which are in a gaseous state under the condition of normal temperature and normal pressure (10°C, atmospheric pressure) are preferred. Further, with a view point of separability and stability after use, carbon dioxide, carbon monoxide, dinitrogen monoxide, ammonia, methane, ethane, propane, butane, and dimethyl ether are preferred. Particularly, since the carbon dioxide has a mild critical temperature and is excellent in safety and stability, it is used particularly preferably.

The sub-critical fluid and super-critical fluid after being used for decreasing β-hydroxypropionic acid (salt) in the acrylate-base polymer may be used again by way of a purification operation (for example, distilling operation, adsorption operation, absorption operation, etc.) or not by way of such purification operation.

In the production method of the adhesive for a dermal patch of the present invention, an entrainer (extraction auxiliary agent) may also be added optionally to the sub-critical fluid and/or super-critical fluid. Use of the entrainer can improve the dissolution power of β-hydroxypropionic acid(salt) contained in the acrylate-base polymer or improve the selectivity upon separation. The entrainer to be used is not particularly limited and may be selected in view of the impurities as an object, and with a view point of the affinity with the sub-critical fluid/or super-critical fluid to be used, ease of recovery, safety and stability.

The entrainer includes, for example, organic solvents, water, as well as liquid mixtures of organic solvents and water. Specific examples of the organic solvent includes, for example, alcohols of 1 to 30 carbon atoms, ketones of 3 to 30 carbon atoms, ethers of 2 to 30 carbon atoms, acetate esters of 3 to 30 carbon atoms, hydrocarbons of 1 to 30 carbon atoms, carbonates of 3 to 30 carbon atoms, and aromatic hydrocarbons of 6 to 30 carbon atoms.

Specific examples include, for example, alcohols such as methanol, ethanol, 1-propanol, 2-propanol, 1-butanol, 2-butanol, i-butyl alcohol, t-butyl alcohol, 1-pentanol, 2-pentanol, 3-pentanol, isoamyl alcohol, 4-methyl-2-pentanol, 1-hexanol, 2-hexanol, 3-hexanol, cyclohexanol, 1-heptanol, 2-heptanol and 1-octanol; ketones such as acetone, methyl ethyl ketone, methyl isobutyl ketone, 2-hexanone, 3-hexanone, 2-pentanone, and 3-pentanone; ethers such as dimethyl ether, diethyl ether and diisopropyl ether; acetate esters such as methyl acetate, ethyl acetate, propyl acetate, butyl acetate, methyl butyrate, ethyl butyrate, propyl butyrate and butyl butyrate; hydrocarbons such as methane, ethane, propane, butane, pentane, hexane, and cyclohexane; carbonates such as ethylene carbonate, dimethylcarbonate, diethylcarbonate, and diphenylcarbonate; aromatic hydrocarbons such as benzene, toluene and xylene.

Among them, in view of the effect as the entrainer and ease of recovery, methanol, ethanol, 1-propanol, 2-propanol, acetone, dimethyl ether, diethyl ether, diisoprpyl ether, methyl acetate, ethyl acetate, methane, ethane, propane, butane and pentane are preferred and, particularly, methanol, ethanol, 1-popanol, 2-propanol, acetone, and dimethyl ether are more preferred.

The entrainers may be used individually or in combination of two or more thereof. In a case of using plural kinds of them, they may be used simultaneously or used individually and successively.

The entrainer may be used throughout the step of bringing the acrylate-base polymer into contact with the sub-critical fluid and/or super-critical fluid, or may be used in a portion of the step of bringing the acrylate-base polymer into contact with the sub-critical fluid and/or super-critical fluid. Specifically, it may include, for example, (1) a method of contacting the sub-critical fluid and/or super-critical fluid with addition of the entrainer and the acrylate-base copolymer and then contacting the sub-critical fluid and/or super-critical fluid not containing the entrainer and the acrylate-base polymer, or (2) a method of contacting the sub-critical fluid and/or super-critical fluid not containing the entrainer and the acrylate-base polymer and then contacting the sub-critical fluid and/or super-critical fluid with addition of the entrainer and the acrylate-base polymer, etc.

The entrainer after use can be used again by way of a purification operation (for example, distilling operation, adsorption operation, etc.). Alternatively, it may be used again without by way of the purifying operation.

In a case of using the entrainer, the addition amount thereof is, preferably, from 0.0001 to 100 mass parts, more preferably, from 0.001 to 30 mass parts and, particularly preferably, from 0.01 to 10 mass parts and, most preferably, from 0.1 to 3 mass parts, based on 100 mass parts of the sub-critical fluid and/or super-critical fluid. In a case of using plural kinds of entrainers, the addition amount referred to herein is a total mass for them. In a case where the amount of using the entrainer in each of the ranges described above is appropriately decreased as each of the upper limits recovery load can be decreased. Further, in case where it is properly increased as each of the lower limits, the extraction effect can be provided sufficiently.

Before bringing the acrylate-base polymer into contact with the sub-critical fluid and/or super-critical fluid, a pre-treatment operation may optionally be applied to the acrylate-base polymer.

As the pretreatment operation, the grain size of the acrylate-type polymer in a solid state can be controlled continuously or batchwise by using a dry or wet type pulverizing apparatus. Further, when classification is necessary, classification may be conducted continuously or batchwise by using a dry or wet type classifying apparatus. In this case, an apparatus having both a pulverization mechanism and a classification mechanism together may also be used. On the contrary, in a case where the grain size of the acrylate-base polymer is excessively small to cause difficulty in the operation, a pelleting operation of a self-sustaining pelleting system or forced pelleting system may also be conducted.

Further, as the pretreatment operation, a preliminary cleaning operation for the acrylate-base polymer of containing much β-hydroxy propionic acid (salt) content may be conducted. For example, cleaning may be conducted by using a cleaning solvent which is liquid or solid under normal temperature and normal pressure condition (for example, water, methanol, etc.) under such a temperature condition as not causing the denaturation of the acrylate-base polymer. The impurities (also including residual cleaning solvent) in the acrylate-base polymer after preliminary cleaning can be separated from the acrylate-base polymer by using the sub-critical fluid and/or super-critical fluid in accordance with the method of the present invention. Further, also the state of phase of the acrylate-base polymer (solid state, liquid state, gel state, etc.) before and after the conduction of the preliminary cleaning operation is not particularly limited, but powdered state is preferred.

In a case of contacting the acrylate-base polymer and the sub-critical fluid and/or super-critical fluid, they may be contacted for an appropriate period of time irrespective of the absence or presence of the entrainer, by selecting the temperature and pressure condition under which at least a portion of β-hydroxypropionic acid (salt) contained in the acrylate-base polymer is dissolved.

The temperature condition is, preferably, from 0.2 to 10 times the critical temperature, more preferably, from 0.8 to 5 times the critical temperature and, particularly preferably, from 0.9 to 2 times the critical temperature of the entrainer. For each of the ranges, the upper limit value has a meaning in view of suppressing the denaturation of the acrylate-base polymer etc. while the lower limit value has a meaning in view of reducing the load required for cooling, etc.

The pressure condition is, preferably, from 0.2 to 60 times the critical pressure, more preferably, from 0.5 to 10 times the critical pressure and, particularly preferably, from 0.8 to 5 times the critical pressure, irrespective of the absence or presence of the entrainer. For each of the ranges, the upper limit value has a meaning for avoiding the necessity of high pressure resistance which makes the apparatus design difficult, while the lower limit value has a meaning in view of the solubility of β-hydroxypropionic acid (salt) to the sub-critical fluid and/or super-critical fluid.

The time of contact is, usually, 50 hours or less, preferably, 10 hours or less, more preferably, 2 hours or less, particularly preferably, 30 min or less and most preferably, 1 min or less. For each of the ranges, the upper limit value has a meaning for avoiding the necessity of a large-sized apparatus which makes the apparatus design difficult, while the lower limit value has a meaning with a view point of sufficient removal of β-hydroxypropionic acid (salt) from the acrylate-base polymer.

The amount of the sub-critical fluid and/or the super-critical fluid to be used is not particularly limited. It may be such an amount as capable of obtaining an aimed acrylate-base polymer of high purity in accordance with the concentration of β-hydroxypropionic acid (salt) in the acrylate-base polymer. The specific amount of use is, preferably, 500 mass parts or less, more preferably, 300 mass parts or less and, particularly preferably, 100 mass parts or less based on 1 mass part of the acrylate-base polymer. For each of the ranges, the upper limit value has a meaning in view of the decrease of the recovery load. etc., while the lower limit value has a meaning for providing a sufficient effect of removing β-hydroxypropionic acid (salt).

The operation of contacting the acrylate-base polymer and the sub-critical fluid and/or super-critical fluid may be any of continuous operation, semi-batchwise operation, or batchwise operation. It is also preferred to conduct continuous multi-stage extraction operation thereby contacting the acrylate-base polymer and the sub-critical fluid and/or super-critical fluid in a counter current manner or parallel current manner.

The apparatus for contacting the acrylate polymer and the sub-critical fluid and/or super-critical fluid is not particularly limited. A preferred apparatus may be selected in accordance with the property of the acrylate-base polymer. Specifically, apparatus, for example, used in a liquid-liquid extraction operation and solid-liquid extraction operation, etc. may also be adopted. More specifically, they include, for example, stirring tank, fixed bed type apparatus, transfer bed type apparatus, kneading apparatus, single screw or twin screw kneader, etc. Further, the state of phase (solid state, liquid state, gel state, etc.) of the acrylate-base polymer before and after contact with the sub-critical fluid and/or super-critical fluid is not particularly limited.

In the production method of the adhesive for a dermal patch of the present invention, the treatment after contact of the acrylate-base polymer and the sub-critical fluid and/or super-critical fluid is not particularly limited.

For example, the acrylate-base polymer, impurities, entrainer and sub-critical fluid and/or super-critical fluid may be separated into desired ingredients and/or groups of ingredients. The specific separation method includes, for example, a method of setting the conditions such that at least a portion of the acrylate-base polymer, β-hydroxypropionic acid (salt), impurities other than β-hydroxypropionic acid (salt), entrainer, and sub-critical fluid and/or super-critical fluid is substantially separated by the control for the temperature and/or pressure. The temperature and/or pressure condition may be set for plural stages. At least a portion of the separated ingredients and/or ingredient groups may be used again. Further, separating operation (for example, distilling operation, adsorbing operation, etc.) may be applied before re-use.

The thus obtained adhesive for a dermal patch comprising an acrylate-base polymer may be used in this state as it is, or grain size may be controlled by further applying pulverization or pelleting. While the aimed grain size may vary depending on the kind of the acrylate-base polymer, it is usually from 10 to 2000 µm, more preferably, from 50 to 1000 µm and, most preferably, from 150 to 500 µm or so in average particle size.

Since the adhesive for the dermal patch obtained according to the present invention has excellent properties, it can be applied to various application uses as described below.
(I) Medicines: for example, preparations for percutaneous absorption, preparations for permucosal absorption.
(II) Medical aids: for example, cooling agent for affected part upon thermogenesis, wound healing medicines, therapeutical pad, surgical liquid absorber, burn healing agent.
(III) Cosmetics, Quasi-drugs: for example, face mask, packing agent, sun-turn healing products, acne healing products.
(IV) Miscellaneous goods: for example, heat removing cooling sheet.

The adhesive for the dermal patch produced according to the invention is used, preferably, within a range from 0.1 to 20 mass% and, more preferably, within a range from 1% to 10% based on the entire amount of the composition when used as the dermal patch. In a case where the amount used is less than 0.1%, syneresis liquid releases from a gelled body to make the adhesive layer not homogenous. On the other hand, if the amount used exceeds 20%, the viscosity of the sol upon molding increases, and the molding or mixing with other ingredients becomes difficult.

Further, upon shaping the adhesive for the dermal patch produced in the present invention into the dermal patch, an aluminum compound is added as a crosslinker with an aim of maintaining the shape retainability of the adhesive layer or preventing "glue residue" to skins.

The adhesiveness can be controlled optionally by changing the amount of the aluminum compound. The aluminum compound is preferably added within a range from 0.01 to 20% and, more preferably, within a range from 0.1 to 10% based on the entire amount of the composition. In a case where the addition amount is less than 0.01%, crosslinking becomes insufficient to cause webbing to the base agent. If it exceeds 20%, gel is hardened excessively to deteriorate the adhesiveness of the composition.

The aluminum compound includes, for example, aluminum chloride, potassium alum, ammonium alum, aluminum nitrate, aluminum sulfate, EDTA-aluminum, aluminum hydroxide, sodium hydrogen carbonate coprecipitates (for example, "KUMULITE", etc., manufactured by Kyowa Chemical Industry Co. Ltd.), synthetic aluminum silicate, aluminum stearate, aluminum allantoinate, synthetic hydrotalcite (for example, "ALCAMAC", "ALCAMIZER", "KYOWARD" etc. manufactured by Kyowa Chemical Industry Co. Ltd.), alumina hydroxide-magnesium (for example, "SANALMIN", etc. manufactured by Kyowa Chemical Industry Co. Ltd.), aluminum hydroxide (for example, "Dried Aluminum Hydroxide Gel S-100", etc. manufactured by Kyowa Chemical Industry Co. Ltd.), aluminum acetate, dihydroxyaluminum amino acetate (for example, "GLYCINAL" manufactured by Kyowa Chemical Industry Co. Ltd., etc.), kaolin, magnesium aluminometasilicate (for example, "NEUSILIN", manufactured by Fuji Chemical Industry Co, Ltd.), and magnesium aluminosilicate. The aluminum compounds may be either water soluble or sparingly soluble. These aluminum compounds may be used individually or in combination of two or more thereof and the combined use of the water soluble aluminum compound and the less soluble aluminum compound can proceed initial crosslinking with the former and subsequent crosslinking with the latter and an adhesive layer excellent in the shape retainability can be obtained in a short time.

Other crosslinkers may also be added. Specific examples include, for example, inorganic acid salts such as of calcium, tin, iron, magnesium, manganese, zinc, and barium (for example, calcium chloride, magnesium chloride, iron alum, ferric sulfate, magnesium sulfate, EDTA-calcium, EDTA-magnesium, stannous chloride, calcium carbonate, calcium phosphate, calcium hydrogen phosphate, magnesium carbonate, barium sulfate, magnesium silicate, magnesium stearate, magnesium citrate), hydroxides (for example, calcium hydroxide, barium hydroxide, magnesium hydroxide (for example, "KISUMA", etc. manufactured by Kyowa Chemical Industry Co. Ltd.), ferric hydroxide, stannaous hydroxide, etc.), oxides (for example, magnesium oxide (for example, "KYOWAMAG", "MAGSARAT", etc. manufactured by Kyowa Chemical Industry Co. Ltd.), formaldehyde, and epoxy compounds such as ethylene glycol diglycidyl ether, glycerin diglycidiyl ether, polyethylene glycol diglycidyl ether, propylene glycol diglycidyl ether, polypropylene glycol diglycidyl ether, etc. The crosslinking agents may be used individually in combination of two or more thereof.

Water is added for improving the solubility of the acrylate-base polymer and providing the viscosity. The addition amount is from 1 to 99.89%. In a case where it is less than 1%, since the effect of increasing the viscosity of the acrylate-base polymer is lowered, this causes a so-called "glue residue" phenomenon in which the polymer remains on release paper or skin surface to which the dermal patch is applied, or so-called "back through" phenomenon in which the adhesive for the dermal patch permeates out through the support. On the other hand, in a case where the amount added is more than 99.89%, solubility of chemicals in the composition is worsened and the diffusion rate of medicines is lowered to deteriorate the absorption to skins.

Further, as the controller for the cross-linking reaction rate, organic acids, organic acid salts, and organic bases having a chelating or coordinating ability to metal ions such as tartaric acid, citric acid, lactic acid, glycolic acid, malic acid, salicylic acid, fumaric acid, methane sulfonic acid, maleic acid, acetic acid, disodium-EDTA, urea, triethylamine and ammonia, as well as inorganic acids such as hydrochloric acid, phosphoric acid, sulfuric acid, nitric acid, and hydrobromic acid can be used. Further, with an aim of improving the solubility and the activity of medicines in the adhesive for the dermal patch and improving the migration thereof into skins, a polyhydric alcohol may be blended. The polyhydric alcohols includes, for example, ethylene glyocol, propylene glycol, 1,3-butylene glycol, ethylene glycol monobutyl ether, diethylene glycol, triethylene glycol, 1,4-butylene glycol (dihydric alcohol), glycerin, trioxyisobutane (trihydric alcohol), erythritol, pentaerythritol (tetrahydric alcohol), xylitol, adnitol (pentahydric alcohol), allodulcitol, sorbitol, sorbitol solution, mannitol (hexahydric alcohol), polyglycerin and dipropylene glycol. Among these, glycerin is particularly preferred in view of the safety and the affinity with the acrylate-base polymer. Polyhydric alcohols may be used individually or in combination of two or more thereof.

Other solvents than the polyhydric alcohols may also be added. The solvents include organic solvents miscible with water, for example, monohydric alcohols such as methanol, ethanol, propanol, benzyl alcohol, phenetyl alcohol, isopropyl alcohol, isobutyl alcohol, hexyl alcohol, 2-ethylhexanol, cyclohexanol, octyl alcohol, butanol, and pentanol, ketones such as acetone and methyl ethyl ketone, cellosolve, dioxane, dimethylformamide, N-methylpyrrolidone, and dimethyl sulfoxide, as well as organic solvents immiscible with water such as ethyl acetate and crotamiton.

Various medicines can be medicated by using the adhesive for the dermal patch produced according to the present invention and typical examples are shown below.
(a) corticosteroids: hydrocortisone, predonisone, becromethasone propionate, flumethasone, triamcinolone, triamcinolone acetonide, fluocinolone, fluocinolone acetonide, fluocinolone acetonide acetate, and clobetasol propionate.
(b) Anti-inflammatory analgesics: salicylic acid, glycol salicylate, methyl salicylate, 1-menthol, camphor, sulindac, sodium trimethine, naproxen, fenbufen, piroxicam, triamcinolone, hydrocortisone acetate, indomethacin, ketoprofen, acetoaminophen, mefenamic acid, flufenamic acid, ibufenac, loxoprofen, tiaprofen, pranoprofen, diclofenac, sodium dichlofenac, alclofenac, lornoxicam, oxyphenbutazone, ibupforen, felbinac, ketorolac, bermoprofen, nabumetone, naproxen, flurbiprofen, fluocinonide, clobetasol propionate, COX-2 inhibitor (etodolac, celecoxib, rofecoxib, nimesulide, meloxicam, etc.), etc.
(c) Antifungals: clotrimazole, tolnaphtate, econazole nitrate, omoconazole nitrate, tioconazole nitrate, ketoconazole nitrate, miconazole nitrate, isoconazole nitrate, sulconazole nitrate, pyrrolnitrin, pimafucin, undecylenic acid, salicylic acid, siccanin, nystatin, exalamide, phenyliodoundecynoate, thiantol, ciclopirox olamine, haloprogin, tricomycin, variotin, pentamycin, amphotericin B, etc.
(d) Antihistamines: antibiotics such as tetracycline hydrochloride, diphenhydramine hydrochloride, chlorpheniramine, diphenyl imidazol and chloramphenicol, diphenhydramine, chlorpheniramine maleate, etc.
(e) Hypnotic sedatives: Phenobarbital, amobarbital, cyclobarbital, lorazepam, haloperidol, etc.
(f) Tranquilizers: fluphenazine, theoridazine, diazepam, flunitrazepam, chlorpromazine, etc.
(g) Antihypertensives: clonidine, clonidine hydrochloride, pindolol, propranol, propranol hydrochloride, bupranolol, indenolol, bucumolol, nifedipine, etc.
(h) Hypotensive diuretics: hydrothiazide, cyclopentiazide, etc.
(i) Antibiotics: penicillin, tetracycline, oxytetracycline, fradiomycin sulfate, erythromycin, chloramphenicol, etc.
(j) Anesthetics: lidocaine, benzocaine, ethyl aminobenzoate, dibucaine, etc.
(k) Antibacterial substances: benzalkonium chloride, nitrofurazone, nystatin, acetosulfamine, clotrimazole, etc.
(l) Vitamins: vitamin A, ergocalciferol, cholecalciferol, octotiamine, riboflavin butyrate, etc.
(m) Antiepileptics: nitrazepam, meprobamate, clonazepam, etc.
(n) Coronary vasodilators: nitroglycerin, nitroglycol, isosorbidinitrate, erythritol tetranitrate, pentaerythritol tetranitrate, propatylnitrate, etc.
(o) Antihistamines: diphenhydramine hydrochloride, chlorpheniramine, diphenylimidazole, etc.
(p) Antitussives: dextromethorphan, terbutaline, ephedrine, ephedrine hydrochloride, etc
(q) Sex hormones: Progesterone, estradiol, etc.
(r) Antidepressants: doxepin etc.
(s) Antianginals: antiperspirants such as diethylamide, camphor, nitroglycerin, isosorbide nitrate, etc.
(t) anesthetic analgesics: morphine hydrochloride, morphine ethyl hydrochloride, morphine sulfate, cocaine hydrochloride, pethidine hydrochloride, codeine phosphate, dihydrocodeine phosphate, fentanyl citrate, Sufentanyl, meperidine hydrochloride, etc.
(u) Crude drugs: cork tree bark, pruni cortex, polygala root, zedoary, german chamomile, trichosanthis semen, licorice root, platycodon, apricot kernel, oriental bezoar, schisandra fruit, Gleditsia japonica, bupleurum root, asarum root, plantago seed, cimicifuga rhizome, senega, atractylodes lancea rhizome, mulberry bark, cloves, citrus unshiu peel, ipecac, Nandinae fructus, fritillariae bulbus, ophiopogon tuber, pinellia tuber, atractylodes rhizome, henbane leaves, Seseleas radix, ephedra, chilly pepper extract, etc.
(v) Others: 5-fluorouracil, dihydro ergotamine, fentanyl, desmopressin, digoxin, metoclopramide, domperidone, scopolamine, scopolamine hydrobromide, as well as animal drugs, hypnotics, therapeutic drugs for circulatory systems, brain metabolism activating drugs, bactericides, enzyme preparations, enzyme inhibitors, living body drugs (polypeptide), drugs for keratosis, dopes, antineoplastic drugs, general anesthetics, antianxiety drugs, asthma /nose allergy drugs, antiparkinsonism drugs, chemotherapy drugs, antiparasitics, anti-protozols, hemostatics, cardiotonics, stimulant/antihypnotic drug, drugs for habitual addiction, herbal drugs, radiopharmaceuticals, drugs for urogenital organ and anus, hypoglycemics, antiulcer drugs, drugs for hairs, sequestering drug, antiperspirants, tranquilizers, anti-blood coagulants, antirheumatics, antigouts and anticoagulants.

Two or more kinds of drugs described above can be used optionally in combination. Preferably, the formulation ratio of the drugs is from 0.01 to 30 mass% and, preferably, 2 to 20 mass% based on the entire mass of the adhesive for the dermal patch.

In the adhesive for the dermal patch produced the present invention, the drug can be incorporated in the stage of solution, in the stage of gel suspension, or after aging for cross-linking reaction. A suitable method is selected in accordance with the physical property of the drug, a part to be medicated, an aimed releasing rate, etc.

Further, adjuvants for promoting the absorption of drugs can be added. The adjuvants include, for example, keratin softening drug such as ethyl alcohol, isopropyl alcohol, n-butanol, 1,3-butane diol, propylene glycol, polyethylene glycol #400, polyethylene glycol #200, polyethylene glycol #300, polyethylene golycol #600, polyethylene golycol #1500, polyethylene golycol #1540, polyethylene golycol #1000, polyethylene golycol #4000, polyethylene golycol #6000, polyethylene golycol #20000, polyethylene golycol #35000, glycerin, crotamiton, benzyl alcohol, phenylethyl alcohol, propylene carbonate, hexyldodecanol, propanol, salicylic acid, allantoin, dimethylsulfoxide, dimethyl acetamide, dimethylformamide, diisopropyl adipate, diethyl sebacate, ethyl laurate, lanolin, eizon, 1-gelanyl azacycloheptane-2-one (GACH), fatty acid dialkylol amide, salicylic acid derivative, urea and sulfur, humectants such as pyrrolidone carboxylic acid, surfactants such as propylene glycol monooleate, polyoxyethylene sorbitane monostearate, sorbitane monostearate and glycerin monostearate, esters such as isopropyl myristate, and diethyl sebacate, higher alcohols such as oleyl alcohol, stearyl alcohol, and lauryl alcohol, fatty acids such as stearic acid, hexanoic acid, nonanoic acid, decanoic acid, dodecanoic acid, tetradecanoic acid, hexadecanoic acid, octadecanoic acid, oleic acid, and linolic acid, terpene-base compounds and surfactants such as menthol, menthone, limonene, pinene, piperitone, terpinene, terpinolene, terpinol, and carveol, auxiliary agents such as allantoin, dimethyl sulfoxide, dimethyl acetamide, dimethylformamide, diisopropyl adipate, diethyl sebacate, ethyl laurate, lanolin, and eizon, and, optionally, cooling agents such as menthol and camphor, oil ingredients such as almond oil, olive oil, camellia oil, persic oil, peppermint oil, sesame oil, soybean oil, mink oil, cotton seed oil, corn oil, safflower oil, palm oil, eucalyptus oil, castor oil, liquid paraffin, vaseline, squalene, squalane, and lanolin, gelling agent such as carboxyvinyl polymer, and neutralizing agents such as diisopropanolamine. They can be blended each alone or by two or more of them. The blending amount is preferably from 0.1 to 5 mass parts based on 100 mass parts of the composition in view of the skin stimulating property, etc.

The dermal patch using the adhesive for the dermal patch according produced in the present invention can be blended with additives exemplified below selectively within a range not deteriorating the adhesive performance with an aim of developing the characteristics further, or improving the fabrication or molding property and the quality, and improvement for the dispersibility and the stability of the medicine in the adhesive layer, etc.
(1) Moisturizers: glycerine, propylene glycol, sorbitol, 1,3-butylene glycol, dl-pyrrolidone carboxylic acid, sodium lactate. etc.
(2) Astringents: citric acid, tartaric acid, lactic acid, aluminum chloride, aluminum sulfate, allantoin chloro hydroxy aluminum, allantoin dihydroxy aluminum, alminium phenol sulfonate, zinc para-phenol sulfate, zinc sulfate, aluminum chlorohydrooxide, etc.
(3) Humectants: polyhydric alcohols such as glycerine, propylene glycol, 1,3-butylene glycol, sorbitol, polygrycerine, polyethylene glycol, and dipropylene glycol, NMF ingredients such as sodium lactate, water soluble polymers such as hyaluronic acid, collagen, mucopolysaccharides, and chondroitin sulfuric acid.
(4) Thickening agents: natural polymers such as gum arabic, traganth gum, locust bean gum, guar gum, ECO gum, karaya gum, agar, starch, carrageenan, alginic acid, alginate (for example, sodium alginate), propylene glycol alginate, dextran, dextrin, amylose, gelatin, collagen, pullulan, pectin, amino pectin, sodium amino pectin semi glyconate, chitin, albumin, and casein, semi-synthetic polymers such as polyglutamic acid, polyaspartic acid, methyl cellulose, ethyl cellulose, propyl cellulose, ethyl methyl cellulose, hydroxy ethyl cellulose, hydroxy propyl cellulose, hydroxy propyl methyl cellulose, carboxymethyl starch, alkali metal carboxymethyl cellulose, alkali metal cellulose sulfate, cellulose graft polymer, crosslinked gelatin, cellulose acetate phthalate, starch-acrylic acid graft polymer, phthalic acid anhydride-modified gelatin, and succinic acid-modified gelatin, and synthetic polymers such as polyvinyl alcohol, polyvinyl pyrrolidone, polyvinyl methyl ether, carboxy vinyl polymer, vinyl pyrrolidone - ethyl acrylate copolymer, vinyl pyrrolidone - styrene copolymer, vinyl pyrrolidone - vinyl acetate copolymer, vinyl acetate - (meth)acrylic acid copolymer, and polyvinyl acetate - crotonic acid copolymer, N-vinyl acetamide type polymer such as N-vinyl acetamide crosslinked product, N-methyl-N-vinylacetamide type polymer such as N-methyl-N-vinyl acetamide crosslinked product, polyvinyl sulfonic acid, N-vinyl acetamide crosslinked product, polyitaconic acid, polyhydroxyethyl acrylate, polyacrylamide, acrylamide-acrylic acid copolymer and styrene-maleic acid anhydride copolymer.
(5) Tackifiers: adhesive materials such as silicone rubber, polyisoprene rubber, styrene block copolymer rubber, acrylic rubber and natural rubber.
(6) Anti-itching agents: camphor, thymol, menthol, polyoxyethylene lauryl ether, antihystamines and ethylamino benzoate.
(7) Keratin softening and peeling agents: sulfur, thioxolone, selenium sulfide, salicylic acid, and resorcin; etc.
(8) Erroneous intake preventive material: red pepper powder, red pepper essence, etc.
(9) Powdery raw materials: montmorillonite anhydrous silicic acid, gypsum, carbon black, diatomaceous earth, red iron oxide, calcium carbonate, hydrotalcite, talc, glass, kaolinite, bentonite, metal soap, aerosil, mica titanium, bismuth oxychloride, fish scales, etc., zinc powder, titanium dioxide, etc.
(10) Oil raw materials: almond oil, olive oil, hardened oil, camellia oil, castor oil, wood wax oil, palm oil, bee wax, whale wax, lanolin, carnauba wax, candelilla wax, liquid paraffin, vaseline, microcrystalline wax, ceresin, squalene, lauric acid, myristic acid, palmitic acid, stearic acid, isostearic acid, oleic acid, lauric alcohol, cetanol, stearyl alcohol, oleyl alcohol, octyl dodecanol, choresterol, hexyl decanol, white sterol, cetyl lactate, isopropyl myristate, hexyl laurate, myristyl myristate, isopropyl palmitate, octyl dodecanol myristate, butyl stearate, cacao oil, jojoba oil, grape seed oil, avocado oil, mink oil, yolk oil, ceresin oil, paraffin wax, behenic acid, isopropyl adipate, octyl dodecyl myristate, octyl dodecyl oleate, and cholesterol oleate, etc.
(11) Surfactants: anionic surfactants such as lauryl sulfate ester salt, polyoxyethylene alkyl ether sulfate salt, alkylbenzene sulfonate salt, polyoxyethylene alkyl ether phosphoric acid, polyoxyethylene alkylphenyl ether phosphoric acid, N-acylamino acid salt, sodium stearate, potassium palmitate, sodium cetyl sulfate, sodium lauryl sulfate, triethanolamine palmitate, sodium polyoxyethylene lauryl phosphate, sodium acyl glutamate, and surfactin, cationic surfactants such as benzalkonium chloride, benzetonium chloride, stearyl trimethyl ammonium chloride, distearyl dimethyl ammonium chloride, and stearyl dimethyl benzyl ammonium chloride, and amphoteric surfactants such as alkyldiaminoethyl glycine hydrochloride, 2-alkyl-N-carboxymethyl-N-hydroxyethyl imidazolinium betain, lauryl dimethylamino acetic acid betain, and lecithin, nonionic surfactant such as polyol fatty acid ester, glycerine monostearate, oleophilic glycerine monooleate, ethylene glycol monostearate, propylene glycol monostearate, sorbitan fatty acid ester, polyoxyethylene sorbitan fatty acid ester, polyoxyethylene fatty acid ester, polyoxyethylene alkyl ether, polyoxyethylene alkyl phenol ether, polyoxyethylene sorbitol fatty acid ester, N-acylamino acid ester, sucrose fatty acid ester, alkylolamide fatty acid ester, polyoxyethylanated sterol, polyoxyethylanated lanolin, and polyoxyethylene hardened castor oil.
(12) Coloring agents: yellow iron oxide, red iron oxide, black iron oxide, ultramarine, carbon black, chromium hydroxide, chromium oxide, tar pigment, lake, Red No. 2, Red No. 3, Red No. 102, Red No. 201, Yellow No. 4, Yellow No. 5, Blur No. 1, Blur No. 2, etc.
(13) Perfumes: plant perfumes such as mustered oil, orange oil, pepper oil, jasmine oil, cedar oil, calamus oil, terpine oil, orange flower oil, rose oil, eucalyptus oil, lime oil, lemon oil, Japanese menthal oil and rosemarie oil, animal perfumes such as musk, civet, castoreum, ambergris, hydrocarbon perfumes such as bromostylol, pinene, and limonen, alcoholic perfumes such as benzyl alcohol and 1-menthol, ester type perfumes such as ethyl acetate and methyl salicylate, aldehyde type perfumes such as benzaldehyde, and salicylaldehyde, ketone type perfumes such as camphor, muscone, musk ketone, and 1-menthon, ether type perfumes such as safrol, phenolic perfumes such as thymol, lactone type perfumes, acid type perfumes such as phenyl acetic acid, and nitrogen compound type perfumes such as indole, etc.
(14) UV-ray cut-off agents: benzophenone series such as ASL-24, Cyasorb UV-9, and Uvinul M-40, benzoic acid series such as Salol, azole series such as Tinuvin P, nitrile series such as Uninul N-35, urea series such as Ancour UA, paraamino acid series such as Neo Heliopan Give tan F, 2-hydroxy-4-methoxybenzophenone, octyldimethyl paramino benzoate, and ethyl hexyl paramethoxy cinnamate, salicylic acid series, benzofurane series, cumarine series, and azole series, etc.
(15) Antiseptic sterilizers: acids such as benzoic acid, salicylic acid, dehydro acetic acid, sorbic acid and boric acid, as well as salts thereof, phenols such as phenol, chlorocresol, chloroxylenol, isopropylmethyl phenol, resorcin, o-phenylphenol, p-oxybenzoic acid ester, phenoxy ethanol, thymol, hinokitiol and thioxolon, halogenated bisphenols such as hexachlorophene and 2,4,4'-trichloro-2'-hydroxy diphenyl ether, amide compounds such as trichlorcarbanilide, halocarban and monoethanolamide undecylenate, quaternary ammonium compounds such as benzalkonium chloride, alkyl isoquinolinium bromide, benzetoniume chloride, and cetyl pyridinium chloride, amphoteric surfactants such as lauryl-di(aminoethyl) glycine, 2-pyridinethiol-1-oxide zinc salt, gluconic acid, chlorohexydine, thiram N-trichloromethylthio-4-cyclohexene-1,2-dicarboxyimide, and chlorobutanol, etc.
(16) Antioxidants: nordihydroguaiaretic acid, guaiacum officinalis, propyl gallate, butylhydroxyanisol, dibutylhydroxy toluene (BHT), tocopherol (vitamin E), 2,2'-methylenebis(4-methyl-6-t-butyl) phenol, etc.
(17) Chelating agents: edetate, pyrophosphate, hexametaphosphate, citric acid, tartaric acid, gluconic acid, etc.
(18) UV-ray scattering agents: titanium oxide, kaolin and talc.
(19) pH adjusters: alkalis, for example, alkali metal hydroxides, alkaline earth metal hydroxides, primary, secondary or tertiary alkyl amines, or primary, secondary or tertiary alkanol amines such as sodium hydroxide, potassium hydroxide, calcium hydroxide, magnesium hydroxide, ammonia, aqueous ammonia, triethanol amine, dimethyl amine, diethyl amine, trimethyl amine, triethyl amine, triisopropanol amine, trisodium phosphate, disodium hydrogen phosphate, dipotassium hydrogen phosphate, monoethanol amine, diethanol amine, diisopropanol amine, and polyethanol amine, further, acids such as citric acid, tartaric acid, lactic acid, glucolic acid, hydrochloric acid, nitric acid, malic acid and phosphoric acid, as well as acidic or alkaline polymers are also used including, for example, alginic acid, polyglutamic acid, polyaspartic acid, starch-acrylic acid graft polymer, polyacrylic acid, vinyl acetate - crotonic acid copolymer, vinyl acetate - (meth)acrylic acid copolymer, polyvinyl sulfonic acid, polyitaconic acid, styrene - maleic anhydride copolymer, and acrylamide - acrylic acid copolymer.
(20) Others: stabilizer, filler, preservative, plasticizer, softening agent, deterioration inhibitor, etc.

### Manufacturing method of dermal patch:

In manufacturing a dermal patch by using an adhesive for the dermal patch produced according to the present invention, after mixing respective starting materials, they are cast in a state of sol into an appropriate die in which they are molded by crosslinking, or the gel after crosslinking is prepared directly into various molding products by using an appropriate molding machine, tabulating machine, etc. The starting materials can be mixed, for example, by selectively using, for example, a kneader, co-kneader, kneader ruder, agi-homomixer, planetary mixer, double planetary mixer, etc. appropriately.

For forming the adhesive for dermal patch into a sheet, the adhesive for the dermal patch may be coated in an appropriate amount to one or both of surfaces of a support such as paper, wood material, metal, glass fiber, cloth (flannel, woven fabric, non-woven fabric, etc.), synthetic resin (polyurethane, ethylene vinyl acetate copolymer, polyvinyl chloride, polyester (for example, polyethylene terephthalate), polyolefin (for example, polyethylene and polypropylene), polyamide (for example, nylon 6 and nylon 66), polyvinylidene chloride, polytetrafluoroethylene, etc.), a metal foil such as of aluminum, rubber or cellulose derivative and molded article thereof (for example, a laminate film with plastic film), a sheet (foil) or a tape. For facilitating the preservation of the obtained sheet-like adhesive for the dermal patch, it is preferred to attach a releasing sheet treated with silicone or like other appropriate means to the surface coated with the adhesive for dermal patch, or treat the surface not coated with the adhesive for the dermal patch with silicone or like other appropriate means to form a releasable surface and then roll-up the same such that it overlaps the surface not coated with the gel. As the releasing sheet, a polyethylene film, polypropylene film, releasing paper, cellophane, polyvinyl chloride, polyester, etc. are used.

### BRIEF DESCRIPTION OF THE DRAWINGS

Fig.1 shows the aging change of the gel strength in the crosslinking experiment of the acrylic acid - sodium acrylate copolymer obtained in Example 1 and Comparative Example 1.
Fig.2 shows the aging change of the gel strength in the crosslinking experiment of the sodium polyacrylate obtained in Example 2 and Comparative Example 2.

### BEST MODE FOR CARRYING OUT THE INVENTION

Then, the present invention is described below by referring to Examples. In Examples, the "parts" is "parts by mass"

### Production method of acrylate-base polymer:

### Example 1 (Reference only):

Acrylic acid, sodium acrylate and water were charged in a 2 L of separable flask. The acrylic acid/sodium acrylate ratio was 50/50 (molar ratio). Ammonium persulfate as a polymerization initiator, potassium hydrogen sulfite as a polymerization accelerator and sodium hypophosphite as a chain transfer agent were added thereto and polymerization was conducted at a polymerization starting temperature of 20°C for 10 hours. The obtained agar-like gel was minced, dewatered with ethanol (water content in the mince after ethanol dewatering : 35%), which was dried by hot blow under a nitrogen gas stream at 210°C for whole day.

β-hydroxypropionic acid in the polymer was extracted with an aqueous solution of acetonitrile and quantitatively determined by HPLC., It was subjected to UV detection at 218 nm at a column temperature of 40°C using an 0.1% of aqueous phosphoric acid solution as an eluent and SHODEX KC-811 (manufactured by Showa Denko K.K.) as a column. As a result, β-hydroxypropionic acid was 30 ppm.

### Comparative Example 1:

Acrylic acid, sodium acrylate and water were charged in a 2 L of separable flask. The acrylic acid/sodium acrylate ratio was 50/50 (molar ratio). Ammonium persulfate as a polymerization initiator, potassium hydrogen sulfite as a polymerization accelerator and sodium hypophosphite as a chain transfer agent were added and polymerization was conducted at a polymerization starting temperature of 20°C for 10 hours. The obtained agar-like gel was minced and dried by hot blow under a nitrogen gas stream at 210°C for whole day.

When the content of β-hydroxypropionic acid in the polymer was quantitatively determined in the same manner as described above, it was 7800 ppm.

### Example 2:

Sodium acrylate and water were charged in a 2 L of separable flask. Ammonium persulfate as a polymerization initiator, potassium hydrogen sulfite as a polymerization accelerator and sodium hypophosphite as a chain transfer agent were added thereto and polymerization was conducted at a polymerization starting temperature of 10°C for 15 hours. The obtained agar-like gel was minced and dried by hot blow at 50°C for whole day.

When the content of β-hydroxypropionic acid in the polymer was quantitatively determined in the same manner as described above, it was 110 ppm.

### Comparative Example 2:

Sodium acrylate and water were charged in a 2 L separable flask. Ammonium persulfate as a polymerization initiator, potassium hydrogen sulfite as a polymerization accelerator and sodium hypophosphite as a chain transfer agent were added thereto and polymerization was conducted at a polymerization starting temperature of 10°C for 15 hours. The obtained agar-like gel was minced and dried by hot blow at 200°C for whole day.

When the content of β-hydroxypropionic acid in the polymer was quantitatively determined in the same manner as described above, it was 8600 ppm.

### Example 3:

Sodium acrylate - N-vinyl acetamide copolymer (copolymerization ratio: sodium acrylate/N-vinyl acetamide = 40/60 mass ratio) was polymerized by aqueous polymerization. The obtained agar-like gel (water content : 75%) was dried by hot blow at 200°C for whole day and formed into a powder. Then, the powder was purified by using a super-critical fluid of gaseous carbon dioxide. A super-critical CO₂ extraction apparatus (standard type) manufactured by Showa Tansan Co,. LTD. was used for the purification. The concentration of β-hydroxypropionic acid before purified was 6700 ppm.

As the treating condition, 100 g of the polymer was charged to a 500 mL extractor and 1000 NL of gaseous carbon dioxide was passed for one hour. It was passed only for once under the condition at 40°C, 30 M·Pa. Since the copolymer was a fine powder, gaseous carbon dioxide was passed from above and below the extractor for suppressing the diffusion of the sample in the extractor during pressure rising and the pressure was elevated to 10 M·Pa. At 10 M·Pa or higher, the gaseous carbon dioxide was passed only from the lower path. As the condition for reducing the pressure, it was lowered to 4 M·Pa for 10 to 12 min (500 NL/h to 1000 NL/h) and was lowered from 4 M·Pa to the atmospheric pressure for 5 min (500 NL/h or lower). Expansion of the sample after the treatment was not observed.

When the content of β-hydroxypropionic acid in the polymer was quantitatively determined in the same manner as described above, it was 10 ppm.

### Example 4:

A polyacrylic acid crosslinked product (Carbopol 934, manufactured by NOVEON Inc., powder) was purified by using a gaseous carbonic dioxide super-critical fluid. A super-critical CO₂ extraction apparatus (standard type) of manufactured by Showa Tansan Co,. LTD. was used for the purification. The concentration of β-hydroxypropionic acid before purified was 5600 ppm.

As the treating condition, 30 g of the polymer was charged to a 500 mL of extractor and 1000 NL of gaseous carbon dioxide was passed for 5 hours. Ethanol was added as an entrainer under the condition of 40°C, 15 M·Pa. The gas was passed twice. Since the polymer was a fine powder, gaseous carbon dioxide was passed from above and below the extractor for suppressing the diffusion of the sample in the extractor during pressure rising and the pressure was elevated to 10 M·Pa. At 10 M·Pa or higher, gaseous carbon dioxide was passed only from the lower path. As the condition for reducing the pressure, it was lowered to 4 M·Pa for 10 to 12 min (500 NL/h to 1000 NL/h) and lowered from 4 M·Pa to the atmospheric pressure for 5 min (500 NL/h or lower). Expansion of the sample after the treatment was not observed.

When the content of β-hydroxypropionic acid in the polymer was quantitatively determined in the same manner as described above, it was 350 ppm.

### Crosslinking Experiment 1:

The acrylic acid - sodium acrylate copolymer obtained in Example 1 (0.2% aqueous solution, viscosity : 450 mPa·s) and the acrylic acid - sodium acrylate copolymer obtained in Comparative Example 1 (0.2% aqueous solution, viscosity : 463 mPa·s) were used and the crosslinking experiment was conducted by the following blend and formulation.

### [Blend]

| | |
|---|---|
| Acrylic acid - sodium acrylate copolymer | 16 parts |
| Glycerine | 120 parts |
| Tartaric acid | 0.8 parts |
| Dried aluminum hydroxide gel | 1.6 parts |
| Water | 240 parts |

### [Formulation]

A liquid dispersion of sodium polyacrylate polymer (16 g), glycerine (120 g) and dried aluminum hydroxide gel (1.6 g) was added to a mixed solution of water (240 g) and tartaric acid (0.8 g)and kneaded till they were homogenized. After molding and tightly sealing the obtained sol, it was aged at about 20°C for 10 days. Gel strength was measured as a parameter for evaluating the degree of crosslinking. That is, it was measured according to the principle of a card tension meter by using a digital force gauges (DFG-0.2K manufactured by NIDEC-SHIMPO CORPORATION) and a stand exclusively used therefor. The functional shaft (made of aluminum) was a circular post of 10 mm diameter and 12 mm height, the moving distance was 30 mm and the moving speed was 5 mm/sec. Gelling was conducted by casting in a state of a sol into a sample bottle made of a resin and measured at n = 3.

The correlation between the measured values for the gel strength according to the measuring method described above and a functional test according to finger touch is as shown below.

**Table 1**

| Measured value (gf) | Expression according to the functional test |
|---|---|
| 20 or less | not crosslinked (webbing occurred upon finger touch on gel, with no repulsion) |
| approx. 30 | only slightly crosslinked (webbing occurred upon finger touch on gel, with slight repulsion) |
| approx. 50 | slightly crosslinked (a little extension upon finger touch on gel, with repulsion) |
| approx. 70 | crosslinked (considerable extension upon finger touch on gel, with strong repulsion) |
| 90 or more | considerably crosslinked (with no so remarkable extension even when pulling the gel with fingers) |

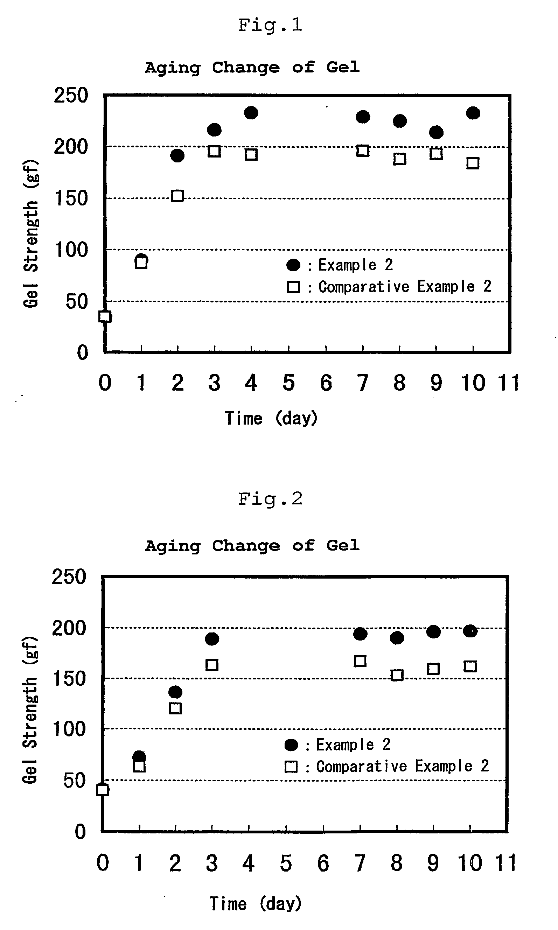

The result is shown in Fig. 1. From Fig. 1, it can be seen that the polymer of Comparative Example 1 with high content of β-hydroxypropionic acid (salt) showed slow gelling rate (progressing degree for the extent of crosslinking).

### Crosslinking Experiment 2:

The sodium polyacrylate obtained in Example 2 (0.2% aqueous solution, viscosity : 560 mPa·s) and the sodium polyacrylate obtained in Comparative Example 2 (0.2% aqueous solution, viscosity : 551 mPa·s) were used and the crosslinking experiment was conducted by the following blend and formulation.

### [Blend]

| | |
|---|---|
| Sodium polyacrylate | 16 parts |
| Glycerine | 120 parts |
| Lactic acid | 1.6 parts |
| Glycinal | 0.4 parts |
| Water | 240 parts |

### [Formulation]

A liquid dispersion of sodium polyacrylate polymer (16 g), glycerine (120 g) and glycinal (0.4 g) was added to and mixed with a liquid mixture of water (240 g) and lactic acid (1.6 g) and kneaded till they were homogenized. After molding and tightly sealing the obtained sol, it was aged at about 20°C for 10 days. The gel strength was measured in the same manner as in Crosslinking Experiment 1. The result is shown in Fig. 2. It can be seen that the polymer of Comparative Example 2 with high content of β-hydroxypropionic acid (salt) showed slow gelling rate (progressing degree for the crosslinking extent).

### INDUSTRIAL APPLICABILITY

Since the acrylate-base polymer produced according to the invention has less content of β-hydroxypropionic acid (salt) and, as a result, crosslinking reaction between the carboxyl groups and aluminum in the polymer can be conducted stably, it is possible to manufacture a dermal patch having stabilized shape retainability, adhesion and percutaneous absorption of medicines.

## Claims

1. A process for producing an adhesive for a dermal patch comprising an acrylate-base polymer containing 5 mol% or more of acrylic acid and/or salt thereof as a monomer unit in which the content of β-hydroxypropionic acid and/or salt thereof is 5,000 ppm or less per solid content of the polymer and an aluminum compound, wherein a drying step of controlling the acrylate-base polymer to a temperature of 40 to 160°C or lower is included.

2. A process for producing an adhesive for a dermal patch comprising an acrylate-base polymer containing 5 mol% or more of acrylic acid and/or salt thereof as a monomer unit in which the content of β-hydroxypropionic acid and/or salt thereof is 5,000 ppm or less per solid content of the polymer and an aluminum compound, wherein a step of drying the acrylate-base polymer is included, and the water content in the acrylate-base polymer before drying step is controlled to 40% or less.

3. The process for producing an adhesive for a dermal patch according to claim 2, wherein a dewatering step of dewatering the acrylate-base polymer previously with a water soluble organic solvent before the drying step is included.

4. The process for producing an adhesive for a dermal patch according to any one of claims 1 to 3, wherein the step of reducing the content of β-hydroxypropionic acid and/or salt thereof by bringing the same into contact with a sub-critical fluid and/or super-critical fluid is included.

5. The process for producing an adhesive for a dermal patch according to claim 4, wherein the sub-critical fluid and/or super-critical fluid is carbon dioxide.

6. The process for producing an adhesive for a dermal patch according to claim 1 or 2, wherein an oxycarboxylic acid other than β-hydroxypropionic acid and an aluminum compound are blended after drying.

7. The process for producing an adhesive for a dermal patch according to claim 1 or 2, wherein the acrylate-base polymer is sodium acrylate -N-vinyl acetamide copolymer.

8. The process for producing an adhesive for a dermal patch according to claim 1 or 2, wherein the acrylate-base polymer has the repetitive unit represented by the formulae (1), (2) and (3): 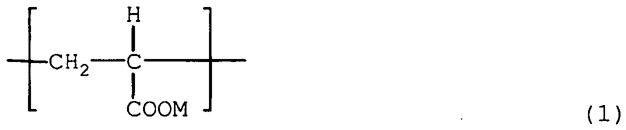 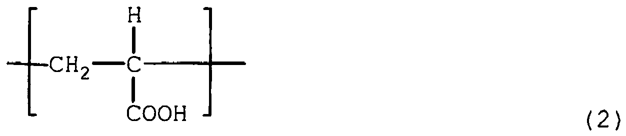
-X- (3)
wherein M represents NH₄⁺ or an alkali metal, and X represents N-vinylacetamide, N-methyl-N-vinylacetamide, N-vinyl pyrrolidone, methacrylic acid (salt), methoxy polyethyleneglycol(meth)-acrylate, N,N- dimethylaminoethyl(meth)acrylate, or acrylamide unsaturated monomer, with a ratio of (1)/(2) being in a range from 100/0 to 0/100 (molar ratio) and total of (1) and (2)/(3) being in a range from 100/0 to 5/95.

9. The process for producing an adhesive for a dermal patch according to claim 8, wherein M is sodium the ratio of the repetitive unit (1) and (2) is within a range of: (1)/(2)=20/80 to 80/20 (molar ratio) and the repetitive unit (3) is not contained.

10. The process for producing an adhesive for a dermal patch according to claim 1 or 2, wherein the acrylate-base polymer is intra-molecularly crosslinked.

11. A process for producing an adhesive used for a dermal patch comprising an acrylate-base polymer containing 5 mol% or more of acrylic acid and/or salt thereof as a monomer unit in which the content of β-hydroxypropionic acid and/or salt thereof is 5,000 ppm or less per solid content of the polymer, which includes:
a) a step of preparing a polymer containing 5 mol% or more of acrylic acid and/or salt thereof as a monomer unit;
b) a step of controlling a β-hydroxypropionic acid content in the polymer to 5,000 ppm or less;
c) a step of adding to the polymer water, a controller for the cross-linking reaction rate and a crosslinker containing an aluminium compound; and
d) a step of crosslinking the above mixture.

12. The process for producing an adhesive used for a dermal patch according to claim 11, wherein the controller for cross-linking reaction rate is oxycarboxylic acid.

13. The process for producing an adhesive used for a dermal patch according to claim 12, wherein the oxycarboxylic acid is other than P-hydroxypropionic acid.

14. The process for producing an adhesive used for a dermal patch according to claim 11, wherein polyhydric alcohol is further blended in step c).

## Patentansprüche

1. Verfahren zum Herstellen eines Klebstoffs für ein Hautpflaster, der ein Polymer auf Acrylatbasis, das 5 mol-% oder mehr Acrylsäure und/oder eines Salzes davon als Monomereinheit enthält, wobei der Gehalt an β-Hydroxypropionsäure und/oder eines Salzes davon 5000 ppm oder weniger in Bezug auf den Feststoffgehalt des Polymers ist, und eine Aluminiumverbindung umfasst, wobei eine Trocknungsstufe durchgeführt wird, in der das Polymer auf Acrylatbasis auf eine Temperatur von 40 bis 160°C oder niedriger kontrolliert wird.

2. Verfahren zum Herstellen eines Klebstoffs für ein Hautpflaster, der ein Polymer auf Acrylatbasis, das 5 mol-% oder mehr Acrylsäure und/oder eines Salzes davon als eine Monomereinheit enthält, wobei der Gehalt an β-Hydroxypropionsäure und/oder eines Salzes davon 5000 ppm oder weniger in Bezug auf den Feststoffgehalt des Polymers ist, und eine Aluminiumverbindung umfasst, wobei eine Stufe durchgeführt wird, in der das Polymer auf Acrylatbasis getrocknet wird und der Wassergehalt in dem Polymer auf Acrylatbasis vor der Trocknungsstufe auf 40 % oder weniger kontrolliert wird.

3. Verfahren zum Herstellen eines Klebstoffs für ein Hautpflaster nach Anspruch 2, wobei eine Entwässerungsstufe umfasst ist, in der das Polymer auf Acrylatbasis vor der Trocknungsstufe mit einem wasserlöslichen organischen Lösungsmittel entwässert wird.

4. Verfahren zum Herstellen eines Klebstoffs für ein Hautpflaster nach einem der Ansprüche 1 bis 3, wobei die Stufe umfasst ist, in der der Gehalt an β-Hydroxypropionsäure und/oder eines Salzes davon verringert wird, indem sie bzw. es mit einem subkritischen Fluid und/oder einem superkritischen Fluid kontaktiert wird.

5. Verfahren zum Herstellen eines Klebstoffs für ein Hautpflaster nach Anspruch 4, wobei das subkritische Fluid und/oder das superkritische Fluid Kohlendioxid ist.

6. Verfahren zum Herstellen eines Klebstoffs für ein Hautpflaster nach Anspruch 1 oder 2, wobei eine Oxycarbonsäure, die nicht β-Hydroxypropionsäure ist, und eine Aluminiumverbindung nach dem Trocknen vermischt werden.

7. Verfahren zum Herstellen eines Klebstoffs für ein Hautpflaster nach Anspruch 1 oder 2, wobei das Polymer auf Acrylatbasis Natriumacrylat-N-vinylacetamidcopolymer ist.

8. Verfahren zum Herstellen eines Klebstoffs für ein Hautpflaster nach Anspruch 1 oder 2, wobei das Polymer auf Acrylatbasis die Wiederholungseinheit der Formeln (1), (2) und (3) aufweist: 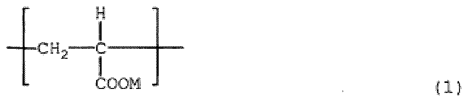 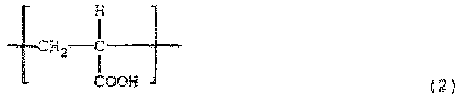
-X- (3)
worin M NH₄⁺ oder ein Alkalimetall darstellt und X N-Vinylacetamid, N-Methyl-N-vinylacetamid, N-Vinylpyrrolidon, Methacrylsäure (Salz), Methoxypolyethylenglycol(meth)-acrylat, N,N-Dimethylaminoethyl(meth)acrylat oder ungesättigtes Acrylamidmonomer darstellt, wobei das Verhältnis von (1)/(2) im Bereich von 100/0 bis 0/100 (Molverhältnis) ist und die Summe von (1) und (2)/(3) im Bereich von 100/0 zu 5/95 ist.

9. Verfahren zum Herstellen eines Klebstoffs für ein Hautpflaster nach Anspruch 8, wobei M Natrium ist, das Verhältnis der Wiederholungseinheit (1) und (2) im Bereich von (1)/(2) = 20/80 bis 80/20 (Molverhältnis) ist und die Wiederholungseinheit (3) nicht enthalten ist.

10. Verfahren zum Herstellen eines Klebstoffs für ein Hautpflaster nach Anspruch 1 oder 2, wobei das Polymer auf Acrylatbasis intramolekular vernetzt ist.

11. Verfahren zum Herstellen eines Klebstoffs für ein Hautpflaster, der ein Polymer auf Acrylatbasis umfasst, das 5 mol-% oder mehr Acrylsäure und/oder eines Salzes davon als Monomereinheit enthält, wobei der Gehalt an β-Hydroxypropionsäure und/oder eines Salzes davon 5000 ppm oder weniger in Bezug auf den Feststoffgehalt des Polymers ist, wobei das Verfahren umfasst:
a) eine Stufe, in der ein Polymer, das 5 mol-% oder mehr Acrylsäure und/oder eines Salzes davon als Monomereinheit enthält;
b) eine Stufe, in der der β-Hydroxypropionsäuregehalt in dem Polymer auf 5000 ppm oder weniger kontrolliert wird;
c) eine Stufe, in der zu dem Polymer Wasser, ein Kontrollmittel für die Vernetzungsreaktionsgeschwindigkeit und ein Vernetzer, der eine Aluminiumverbindung enthält, gegeben wird; und
d) eine Stufe, in der das vorstehende Gemisch vernetzt wird.

12. Verfahren zum Herstellen eines Klebstoffs für ein Hautpflaster nach Anspruch 11, wobei das Kontrollmittel für die Vernetzungsreaktionsgeschwindigkeit Oxycarbonsäure ist.

13. Verfahren zum Herstellen eines Klebstoffs für ein Hautpflaster nach Anspruch 12, wobei die Oxycarbonsäure nicht β-Hydroxypropionsäure ist.

14. Verfahren zum Herstellen eines Klebstoffs für ein Hautpflaster nach Anspruch 11, wobei außerdem mehrwertiger Alkohol in Stufe c) zugemischt wird.

## Revendications

1. Procédé de production d'un adhésif pour un pansement dermique comprenant un polymère à base d'acrylate contenant 5 % en mole ou plus d'acide acrylique et/ou d'un sel de celui-ci comme une unité monomère dans lequel la teneur en acide β-hydroxypropionique et/ou en un sel de celui-ci est de 5 000 ppm ou inférieure par teneur en matière solide du polymère et un composé d'aluminium, dans lequel une étape de séchage consistant à contrôler le polymère à base d'acrylate à une température de 40 à 160°C ou inférieure est incluse.

2. Procédé de production d'un adhésif pour un pansement dermique comprenant un polymère à base d'acrylate contenant 5 % en mole ou plus d'acide acrylique et/ou d'un sel de celui-ci comme une unité monomère dans lequel la teneur en acide β-hydroxypropionique et/ou en un sel de celui-ci est de 5 000 ppm ou inférieure par teneur en matière solide du polymère et un composé d'aluminium, dans lequel une étape de séchage du polymère à base d'acrylate est incluse, et la teneur en eau dans le polymère à base d'acrylate avant l'étape de séchage est contrôlée à 40 % ou inférieure.

3. Procédé de production d'un adhésif pour un pansement dermique selon la revendication 2, dans lequel une étape de déshydratation consistant à déshydrater le polymère à base d'acrylate préalablement avec un solvant organique soluble dans l'eau avant l'étape de séchage est incluse.

4. Procédé de production d'un adhésif pour un pansement dermique selon l'une quelconque des revendications 1 à 3, dans lequel l'étape de réduction de la teneur en acide β-hydroxypropionique et/ou en un sel de celui-ci par mise en contact avec un fluide sous-critique et/ou un fluide super-critique est incluse.

5. Procédé de production d'un adhésif pour un pansement dermique selon la revendication 4, dans lequel le fluide sous-critique et/ou le fluide super-critique est le dioxyde de carbone.

6. Procédé de production d'un adhésif pour un pansement dermique selon la revendication 1 ou 2, dans lequel un acide oxycarboxylique différent de l'acide β-hydroxypropionique et un composé d'aluminium sont combinés après séchage.

7. Procédé de production d'un adhésif pour un pansement dermique selon la revendication 1 ou 2, dans lequel le polymère à base d'acrylate est un copolymère d'acrylate de sodium-N-vinylacétamide.

8. Procédé de production d'un adhésif pour un pansement dermique selon la revendication 1 ou 2, dans lequel le polymère à base d'acrylate présente l'unité répétitive représentée par les formules (1), (2) et (3) : 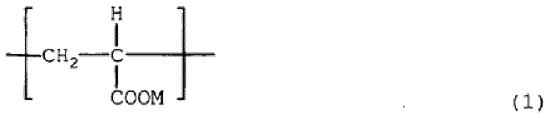 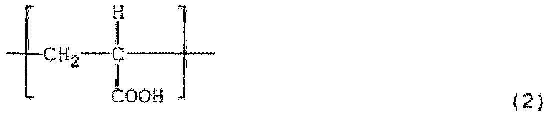
-X- (3)
où M représente NH₄⁺ ou un métal alcalin, et X représente un monomère insaturé de N-vinylacétamide, N-méthyl-N-vinylacétamide, N-vinyl-pyrrolidone, acide méthacrylique (sel), (méth)acrylate de méthoxy-polyéthylèneglycol, (méth)acrylate de N,N-diméthylaminoéthyle, ou acrylamide, avec un rapport de (1)/(2) se trouvant dans un intervalle de 100/0 à 0/100 (rapport molaire) et le total de (1) et (2)/(3) se trouvant dans un intervalle de 100/0 à 5/95.

9. Procédé de production d'un adhésif pour un pansement dermique selon la revendication 8, dans lequel M est le sodium, le rapport de l'unité répétitive (1) et (2) se trouve dans un intervalle de : (1)/(2) = 20/80 à 80/20 (rapport molaire) et l'unité répétitive (3) n'est pas contenue.

10. Procédé de production d'un adhésif pour un pansement dermique selon la revendication 1 ou 2, dans lequel le polymère à base d'acrylate est intra-moléculairement réticulé.

11. Procédé de production d'un adhésif utilisé pour un pansement dermique comprenant un polymère à base d'acrylate contenant 5 % en mole ou plus d'acide acrylique et/ou d'un sel de celui-ci comme une unité monomère dans lequel la teneur en acide β-hydroxypropionique et/ou en un sel de celui-ci est de 5 000 ppm ou inférieure par teneur en matière solide du polymère, lequel comprend :
a) une étape de préparation d'un polymère contenant 5 % en mole ou plus d'acide acrylique et/ou d'un sel de celui-ci comme une unité monomère ;
b) une étape de contrôle d'une teneur en acide β-hydroxy-propionique dans le polymère à 5 000 ppm ou inférieure ;
c) une étape d'addition au polymère d'eau, d'un agent de contrôle pour la vitesse de réaction de réticulation et d'un agent de réticulation contenant un composé d'aluminium ; et
d) une étape de réticulation du mélange ci-dessus.

12. Procédé de production d'un adhésif utilisé pour un pansement dermique selon la revendication 11, dans lequel l'agent de contrôle pour la vitesse de réaction de réticulation est l'acide oxycarboxylique.

13. Procédé de production d'un adhésif utilisé pour un pansement dermique selon la revendication 12, dans lequel l'acide oxycarboxylique est différent de l'acide β-hydroxypropionique.

14. Procédé de production d'un adhésif utilisé pour un pansement dermique selon la revendication 11, dans lequel un alcool polyvalent est de plus combiné dans l'étape c).
